# EUROPEAN PATENT SPECIFICATION

(11) **EP 3 060 298 B1**
(45) Date of publication and mention of the grant of the patent: **01.08.2018**
(21) Application number: 14786903.6
(22) Date of filing: 21.10.2014
(51) Int. Cl.: A61N 5/06, A61N 1/32

(54) **DEVICE FOR COMBINED TREATMENT OF THE VAGINAL CANAL OR OTHER ORIFICES BY MEANS OF LASER RADIATION AND RADIO FREQUENCY CURRENT, AND RELATED APPARATUS**
VORRICHTUNG ZUR KOMBINIERTEN BEHANDLUNG DES VAGINALKANALS ODER ANDERER ÖFFNUNGEN MITTELS LASERSTRAHLUNG UND RADIOFREQUENZSTROM SOWIE ZUGEHÖRIGE VORRICHTUNG
DISPOSITIF POUR TRAITEMENT COMBINÉ DU CANAL VAGINAL OU D'AUTRES ORIFICES AU MOYEN D'UN RAYONNEMENT LASER ET DE COURANT À RADIOFRÉQUENCE, ET APPAREIL ASSOCIÉ

(30) Priority: 22.10.2013 IT FI20130251
(43) Date of publication of application: 31.08.2016
(73) Proprietor: El.En. S.p.A., 50041 Calenzano (Fl) (IT)
(72) Inventor: MODI, Stefano, 50032 Borgo San Lorenzo (FI) (IT); SCORTECCI, Maurizio, 59100 Prato (IT); GALLI, Mauro, 50019 Sesto Fiorentino (FI) (IT); MASOTTI, Leonardo, 50019 Sesto Fiorentino (FI) (IT); CLEMENTI, Gabriele, 50139 Firenze (IT)
(74) Representative: Mannucci, Michele
(86) International application number: PCT/EP2014/072505
(87) International publication number: WO 2015/059120

(56) References cited:
- EP-A1- 2 039 392
- EP-A1- 2 476 460
- WO-A1-00/40294
- WO-A1-03/013653
- WO-A1-2004/105867
- WO-A1-2008/111970
- WO-A1-2011/096006
- WO-A1-2012/037954
- GB-A- 2 129 690
- RU-C1- 2 008 043

## Description

### Technical Field

The present invention relates to the field of electro-medical machines and more in particular to the field of machines and equipment using a laser source for treating the human body.

### Background Art

Various applications of laser radiation are well known for surgical, aesthetic or therapeutic treatment of the human body. In some applications, laser is used instead of a scalpel as cutting tool. In other applications, laser is used to necrotize tumor tissues, to bio-stimulate the growth of particular tissues, for instance cartilaginous tissue for pain treatment and collagen tissue for aesthetic purposes, for instance for wrinkle reduction, rejuvenation, scalp treatment for hair growth purposes, etc.

WO-A-2011096006 and WO-A-2012037954 disclose devices, with a retractor and a system for conveying a laser beam, for treating the tissues of the vaginal canal.

### Summary of the Invention

The present invention concerns an improvement in the device described in WO-A-2011096006, to give this device further functions. The invention is defined by the claims. Essentially, according to an aspect a device is provided for treating the vaginal canal or other orifices, both natural or surgically obtained orifices, by means of a laser beam, the device comprising a retractor for the wall of the vaginal canal or orifice, associated with a system for directing the laser beam towards said wall, and two electrodes for applying a RF current to the tissues of said walls. Preferably, two electrodes associated with the retractor, are preferably parallel to each other.

According to a further aspect an electro-medical apparatus is provided, comprising a laser source, an RF current source, and a device as described above, connected with the laser source and with the RF current source.

In some embodiments, the laser source can be a pulsed laser source. In other embodiments, the laser source can be a continuous laser source. For instance, pulses can be used having a duration between 0.1 and 10 ms and preferably between 0.2 and 2 ms. The laser radiation may have a wavelength comprised between 1,000 nm and 12,000 nm, and preferably equal to 10,600 nm. The laser source may have a power comprised between 2 and 100 W and preferably between 10 and 50 W. By means of scanning mirrors a pulsed treatment pattern can be advantageously generated, wherein the space between subsequent scanning points is between 0 and 5,000 micrometers, preferably between 50 and 5,000 micrometers and more preferably between 200 and 2,000 micrometers.

Further advantageous features and embodiments of the invention are indicated in the appended claims, forming an integral part of the present description, and will be clearly apparent from the description below of an embodiment of the apparatus and the device according to the invention.

### Brief description of the drawings

The invention shall now be more apparent by following the description and accompanying drawing which shows a practical embodiment of the invention. More in particular:
figure 1 is a diagram of the apparatus in one embodiment;
figure 2 is a schematic view of the end part of the articulated arm of the apparatus of figure 1, the scanning system and the retractor;
figure 3 is a side view of the retractor, separated from the remaining part of the apparatus;
figure 4 is a view according to IV-IV of figure 3;
figure 5 shows a section according to V-V of figure 3;
figure 6 and 7 are cross-sections according to VI-VI and VII-VII of figure 3;
figure 8 is an axonometric view of the retractor;
figure 9 is a diagram for fractional treatment of the vaginal tissue;
figure 10A-10C show the path of the laser radiation spot on the reflecting surfaces of the pyramidal mirror in possible uses of the device;
figure 11 is a side view of the retractor showing the electrode system for radio-frequency treatment;
figure 12 is a local section according to XII-XII of figure 11; and
figure 13 shows a side view of a retractor in a modified embodiment.

### Detailed description of an embodiment of the invention

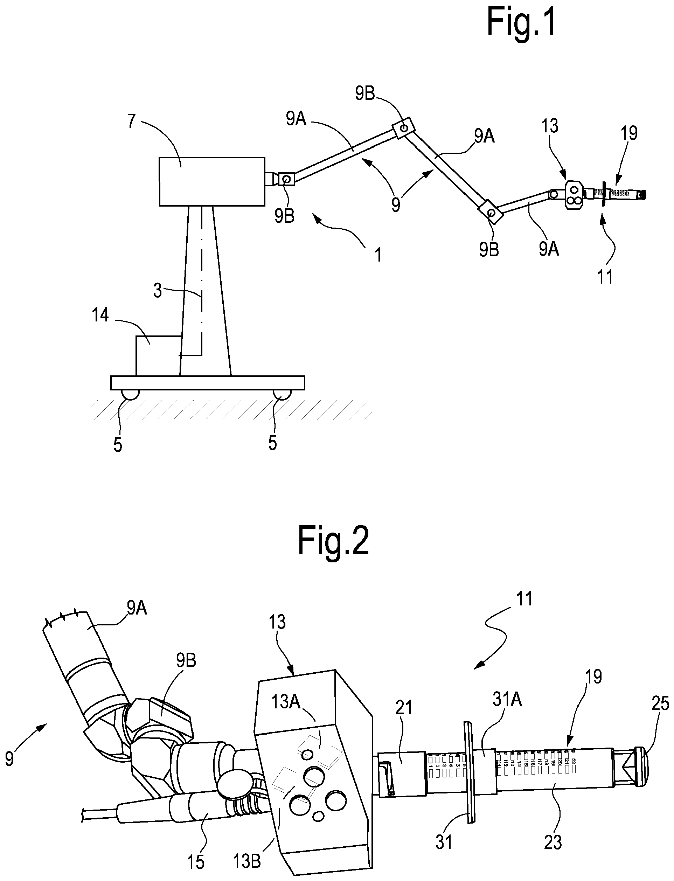

Figure 1 shows a diagram of an apparatus according to the invention. The apparatus, indicated as a whole with number 1, has a support 3 provided for instance with wheels 5 so as to be moved on the floor. The block 7 arranged on the support 3 shows a laser source and an RF current source. The laser radiation is conveyed to a treatment device 11 by means of a waveguide 9. In some embodiments the waveguide 9 is formed by tubular segments 9A joined together by means of known articulated elements 9B to allow the device 11 to be positioned and moved. In the articulated elements scanning mirrors may be provided for conveying the laser beam along consecutive tubular segments. The device 11 is at the terminal end of the waveguide.

An electrical conductor can be applied apply along the articulated arm formed by the tubular segments 9A constituting the waveguide, to convey to the treatment device 11 an RF current coming from the RF current source housed in the block 7.

The device 11 is shown in detail in figure 2. It comprises a box-shaped body forming a housing 13, wherein scanning mirrors are arranged. Two scanning mirrors 13A and 13B are schematically shown with a broken line in the illustrated example. The movement of the mirrors around their axes of rotation is controlled by respective actuators, for instance galvanometers, under the control of a central unit 14, arranged for instance on the support 3 and connected to the device 11 via a cable 15. The control unit 14 is also connected to the laser beam 7 to control the emission thereof. On the box-shaped body forming the housing 13 there may be buttons, capacitive sensors or other interface elements, allowing the user to handle the apparatus and to control the laser emission.

A retractor indicated as a whole with number 19 is associated with the housing 13 of the device 11; this retractor can be advantageously applied to the housing 13 in reversible fashion, so that it is possible to use retractors 19 different in shape and dimension and/or to allow sterilization, or use of disposable retractors 19 for hygiene and asepsis reasons. The retractor 19 can have an outer wall surrounding a hollow interior space. In some embodiments the outer wall is formed by a hollow body, preferably a substantially cylindrical hollow body, such as hollow body 23.

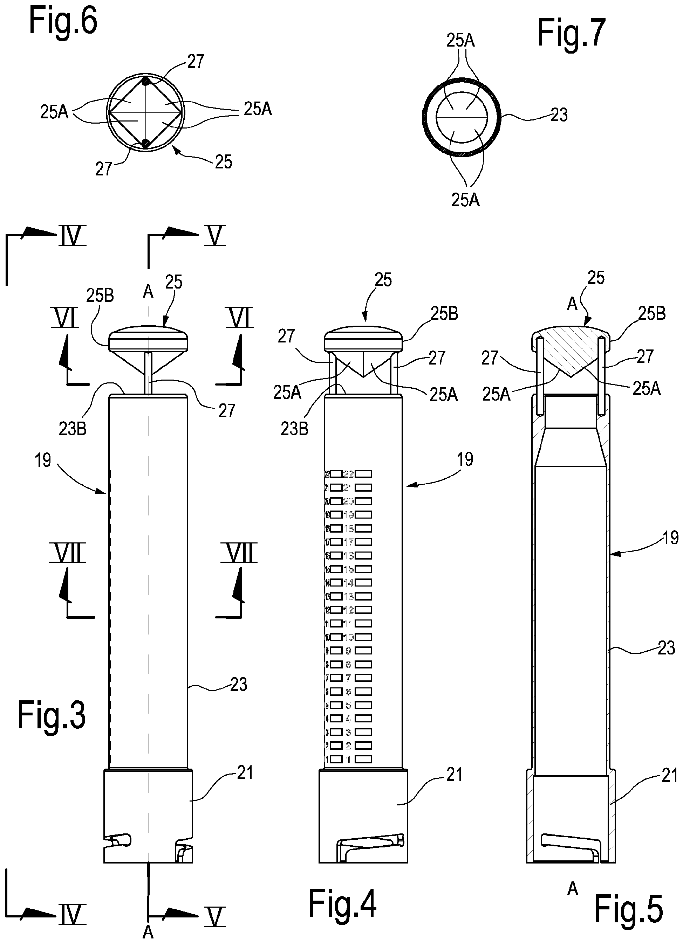

The retractor 19 of the treatment device 11 is shown in detail in figures 3-8.

The retractor 19 can have an elongate shape with a longitudinal axis A-A. The longitudinal axis A-A can be parallel to the intended direction of insertion of the retractor in the vaginal canal or other orifice. The retractor 19 can have a first, proximal end and a second, distal end, aligned along the longitudinal axis A-A. A hollow inner space can be provided between the two ends of the retractor. The inner hollow space can house the laser directing system.

In advantageous embodiments the retractor comprises an outer wall, which can be either solid or in the form of a lattice or network structure, extending around the central, longitudinal axis A-A. In some embodiments the outer wall surrounds the inner hollow space.

The outer wall has at least one side aperture, opening, slit, window or passage, where through laser radiation can pass, the laser radiation being directed towards the side wall of the vaginal canal in the way to be described herein below. A laser directing system is arranged in the hollow space of the retractor. The window, passage, aperture, opening or slit and the laser directing system are arranged such that laser radiation can be directed to exit from the interior of the hollow space through the aperture, slit, or window, in a direction substantially orthogonal to the longitudinal axis A-A of the retractor 19.

In some embodiments the retractor 19 has anchoring members at its distal end, for fixing it to the housing 13 where the scanning mirrors 13A and 13B are located. In the illustrated embodiment the retractor 19 has a bayonet-coupling schematically shown at 21. In other embodiments a different coupling may be provided, for instance a screw coupling. The bayonet-coupling has however specific advantages in terms of quickness in coupling and releasing, as well as of easiness in cleaning the retractor 19 after use, as the bayonet coupling 21 does not have areas forming receptacles for microorganisms.

The retractor 19 may have a substantially cylindrical hollow body 23, as shown in particular in the section of figure 5. At the end opposite the bayonet coupling 21 the hollow body 23 of the retractor 19 carries a mirror, indicated as a whole with number 25. The mirror 25 may be fixed to the tubular body 23 of the retractor 19, for instance by means of bars 27. In other embodiments, the mirror 25 can be housed inside the hollow body, which can be provided with at least one aperture, slit, opening, window or the like, in correspondence of the reflecting surfaces of the mirror, such as to allow laser radiation to pass from the interior of the hollow body 23 towards the surrounding tissue of the vaginal canal, or other orifice, wherein the retractor 19 has been introduced.

In the illustrated embodiment the mirror 25 and the tubular hollow body 23 are connected together by means of two diametrically opposite bars 27. The bars 27 form spacers supporting the mirror 25 at a sufficient distance from the end edge of the retractor 19 to form a nearly annular window, aperture or slit, where through the laser beam passes. The annular slit or opening formed between the end edge, indicated with 23B, of the tubular hollow body 23 and the base, indicated with 25B, of the pyramidal mirror 25, is preferably completely open, so that the laser beam, deviated by means of the reflecting surfaces 25A of the pyramidal mirror 25, propagates in air up to the surface of the surrounding tissue of the vaginal canal in which the retractor 19 is inserted. This allows the application of laser sources with a wavelength that could not pass through closing walls.

For treating the above mentioned dysfunctions of the vaginal tissue the CO₂ laser has been proved to be particularly useful; to its wavelength only toxic materials, such as zinc selenide, are transparent, that are therefore incompatible with this use. It is therefore particularly advantageous to have a free path, i.e. a path in the air, for the radiation reflected by the mirror surfaces 25A of the mirror 25 to treat the wall of the vaginal canal by means of CO₂ laser.

In other embodiments it would however be possible to provide a window closed by means of a material transparent to the wavelength of the laser used, in case nontoxic materials are available that are transparent to the wavelength used. For instance, in the case of treatment with light in or near the visible spectrum, transparent plastics may be used to form walls, where through the path of the reflected laser beam passes. In this case, instead of spacing bars 27, an annular element may be used, made of a material transparent to the laser radiation, interposed between the distal edge 23B of the cylindrical body 23 of the retractor 19 and the base 25B of the mirror 25.

In advantageous embodiments the mirror 25 is shaped like a pyramid with regular polygonal base, for instance and preferably with a square base. In other embodiments, not shown, the mirror 25 may be shaped like a truncated pyramid, also in this case with preferably a regular polygonal base, preferably a square base. It is also possible to use mirrors shaped like a pyramid or a truncated pyramid with different bases, for instance a triangular, pentagonal or hexagonal base. The square shape of the base of the pyramid forming the mirror 25 is particularly advantageous and is currently preferred.

In the illustrated embodiment, the pyramidal mirror 25 with square base has four reflecting surfaces indicated with 25A and formed on the faces of the pyramid. The two bars 27 are advantageously arranged in correspondence of two corners of the square base of the mirror 25 and are therefore arranged substantially on a plane containing two of the fours corners of the pyramid forming the mirror 25.

With the above described arrangement of the reflecting surfaces 25A the movement of the scanning mirrors 13A, 13B allows to perform a particularly comfortable treatment of the tissue of the vaginal canal inside which the device 11 is inserted. In fact, it is sufficient to move the device 11 only in the longitudinal direction, i.e. parallel to its own axis, for instance moving this device 11 gradually outwardly after having inserted it completely inside the vaginal canal. Thanks to the movement of the scanning mirrors controlled by respective galvanometers (known and not shown) the laser beam generated by the source is directed from the mirror faces 25A of the mirror 25 on all the circumferential extension of a given section of the vaginal canal, in correspondence whereof the mirror 25 is positioned by the longitudinal movement according to incremental steps, for instance guided by means of visible marks provided on the retractor portion visible to the operator. On the contrary of what occurs with other known systems, for instance the system described in WO20110960006, it is not necessary to rotate the device 11 inside the vaginal canal, thus making the use of the apparatus easier for the operator and less invasive for the patient. As it will be specified below, in some cases the retractor 19 shall be rotated only once to have a more uniform treatment.

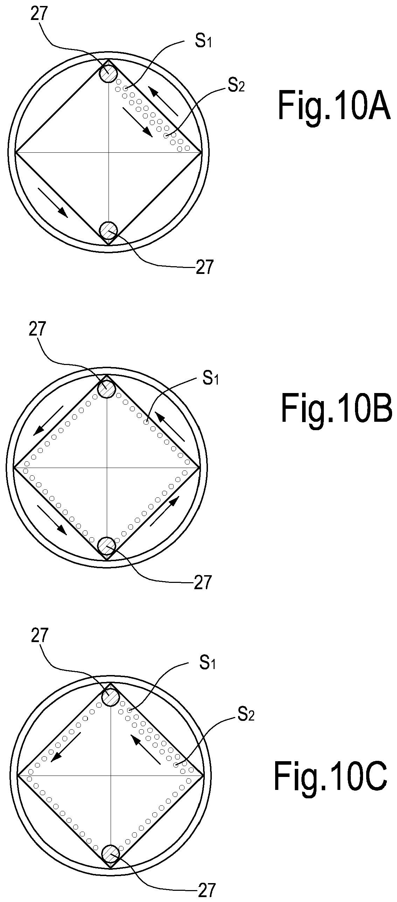

The tissue of the vaginal canal may be irradiated for instance by displacing the laser beam by means of the controlled movement of the mirrors 13A, 13B on each of the four reflecting surfaces 25A of the mirror 25, sequentially. On each face the laser beam can be moved in a direction parallel to the corresponding base edge of the pyramid forming the mirror 25 and gradually from the base towards the vertex or vice versa, so that the laser beam reflected by the mirror surface 25A involves a non-infinitesimal portion of tissue. The laser beam can be then sequentially moved on the remaining three faces of the pyramid to work on each of them. This functioning method is schematically represented in figure 10A, showing, similarly to figure 6, a front view of the pyramidal mirror. The laser beam is controlled by means of the scanning mirrors so as to form a spot moving parallel to the base edge of one of the four faces of the pyramid. In a first phase the laser beam is moved parallel to the base edge and adjacent to it so as to produce a series of spots S1. The laser beam may be actuated in an intermittent pulsed way each time the scanning mirrors 13A, 13B have been positioned to direct the beam in correspondence of one of the spots S1. In correspondence of each spot S1 the beam is deviated, nearly orthogonally to the axis of the retractor 19, towards the side surface of the vaginal canal and nearly orthogonally thereto. Once the row of spots S1 has been completed, the laser beam is moved by means of the scanning mirrors 13A, 13B to form a second row of spots S2, at a greater distance from the base edge, and so on, gradually moving towards the vertex of the pyramid forming the mirror 25. Practically, some lines of spots S1, S2 will be followed, involving a portion of the mirror-like surface 25A near the base. Then, the laser beam is moved on the adjacent face 25A to repeat the process, generating a series of spots on the reflecting surface and, thus, on the tissue of the vaginal canal. The process is repeated on all four faces. As it will be explained below, to avoid non-uniformity due to the edge of the pyramid and the bars 27, the whole process may be repeated rotating the retractor 19, and therefore the pyramidal mirror 25, by an adequate angle, for instance 45°.

In other embodiments the laser beam may be moved in a substantially circular manner to be reflected in sequence by each of the four faces of the pyramidal mirror 25 and moving gradually the laser beam so as to displace at each turn the reflection point of the beam from the base of the pyramid towards the vertex or vice versa, so as to invest an area of sufficient axial dimension of the tissue forming the vaginal canal. This operative mode is illustrated in figures 10B and 10C. In figure 10B the first series of spots S1 is illustrated, created by moving the laser beam along the four base edges of the pyramidal mirror. Once the closed path around the pyramid base has been completed, the beam is moved towards the vertex and along a closed path, forming the spots S2. In figure 10C the first phase is shown of this second movement of the beam with the formation of the spots on one of the four reflecting surfaces 25A of the mirror 25. The process is repeated for a certain number of increasingly shorter paths while moving towards the vertex. Due to the effect of the laser beam reflection by means of the mirror surfaces 25A, spots are therefore generated on the surface of the tissue of the vaginal canal, arranged according to substantially circular trajectories. In this case again, for each axial portion of the retractor 19 in the vaginal canal it is possible to perform the irradiation process twice, rotating the retractor 19 by an angle, for instance by 45°, between one process and the other, to avoid non-uniformity in the treatment.

In other embodiments the laser beam may be controlled so as to make a single movement around the axis of the pyramid-shaped mirror, moving from one face to the other. In this case the distance between the pyramidal mirror reflecting surfaces and the scanning mirror is constant. The trajectory of the laser beam can be increased or decreased to increase or decrease the area of the vaginal canal treated for each position of the retractor.

Advantageously, the four faces forming the reflecting surfaces 25A are inclined by about 45° with respect to the axis A-A of the retractor 19, so that the beam directed approximately parallel to the axis A-A (notwithstanding the slight inclination necessary to bring the beam in an intermediate area of each reflecting face 25A) is reflected in a direction substantially orthogonal to the axis A-A and therefore nearly perpendicularly to the surface of the vaginal canal, inside which the retractor 19 has been inserted.

Controlling the scanning mirrors 13A, 13B as described above, it is therefore possible to treat a "slice" of the vaginal canal wall having a non-negligible dimension in the direction of the axis A-A of the retractor 19. Once this area of the vaginal canal has been treated, the retractor 19 can be moved by one step in axial direction, so as to treat in sequence the subsequent area or slice of vaginal canal.

In advantageous embodiments of the method described above, the laser beam is controlled so that for each position of the retractor 19 the laser beam is controlled to make a trajectory involving a limited portion of the pyramidal or truncated pyramidal mirror, typically in the order of some millimeters, near the base. The axial extension of the mirror portion used is proportional to the "slice" of vaginal canal treated for each position of the retractor 19. The backward or forward movement of the retractor 19 between one phase of the treatment and the next is preferably equal to the width of the treated "slice" so that, once the treatment is completed, all the inner surface of the vaginal canal has been subjected to the laser effect.

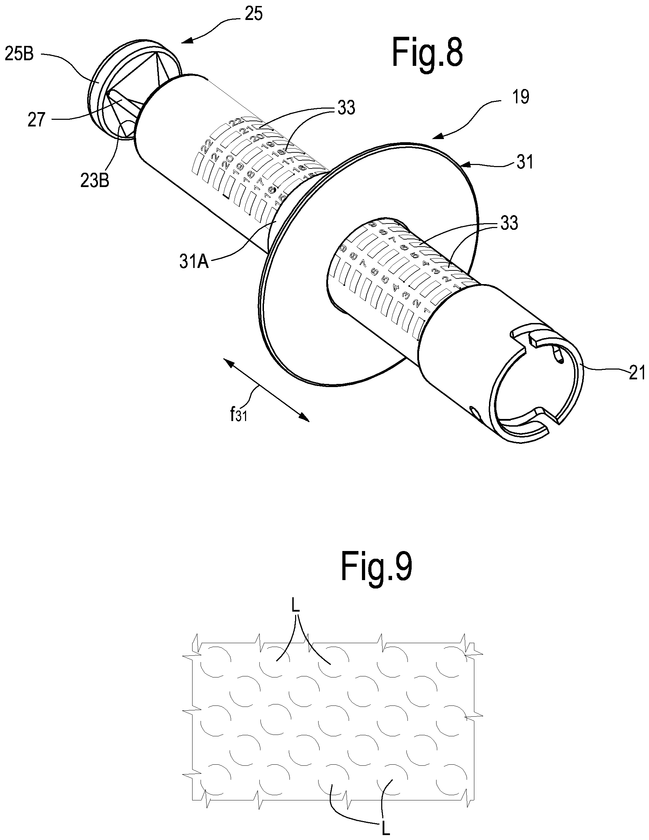

The movement for gradually removing the retractor 19 from the vaginal canal may be better controlled using an element acting as a reference stop on the outside of the vaginal canal, as better shown in figure 8.

To this end, in some embodiments a disc-shaped element 31 is provided, mounted on the outside of the cylindrical body 23 of the retractor 19. The disc-shaped element 31, provided, if necessary, with a suitable collar 31A to increase the support surface on the cylindrical body 23 of the retractor 19, and the cylindrical body 23 of the retractor 19 slide with respect to each other according to the double arrow f31 (figure 8), so as to modify the distance between the mirror 25 and the disc-shaped stop element 31 and consequently to modify the depth of insertion of the retractor in the vaginal canal.

In some advantageous embodiments, reference marks 33 may be provided on the outer surface of the retractor 19, facilitating the operator in positioning and moving gradually the retractor 19 with respect to the disc-shaped stop element 31. The operator can rest the disc-shaped stop element on the vulva structures at the entrance of the vaginal canal and, maintaining it in this position, move the cylindrical body 23 of the retractor 19 parallel to the axial extension of the same retractor 19, gradually extracting it from or inserting it into the vaginal canal, moving it from the end where there is the bayonet coupling 21 towards the opposite end, as the treatment of the vaginal canal tissues proceeds as described above.

The marks 33 allow the operator clearly to identify the position of the retractor 19 with respect to the disc-shaped element 31 so as to treat subsequent "slices" or portions of the vaginal canal gradually and step by step.

The treatment process substantially proceeds as follows: the retractor 19 is positioned, with respect to the disc-shaped stop element 31, in correspondence of the mark closest to the bayonet coupling 21, so that the retractor 19 can be completely inserted inside the vaginal canal until the disc-shaped stop element 31 abuts the patient's body in correspondence of the vaginal canal entrance. The operator actuates the laser and the scanning mirrors 13A, 13B so that, under the control of the programmed electronic control unit, the laser beam treats the whole surface of the vaginal canal that can be achieved moving the laser beam along the mirror surfaces 25A of the mirror 25 from the base towards the vertex of the same mirror or vice versa.

Once the treatment has been completed, the operator partially removes the retractor 19 extracting it by one step, so that the disc-shaped element 31 is aligned with the subsequent mark. The distance between two adjacent marks, in the order of some millimeters, corresponds to the dimension in axial direction of the vaginal canal area that can be treated in a single phase, before moving the retractor.

The operation is repeated for the different positions of the retractor 19 with respect to the disc-shaped stop element 31 up to the entrance of the vaginal canal.

In other embodiments the operator can act reversely, treating firstly the area closest to the vaginal canal entrance and gradually continuing towards the inside of the canal.

In some embodiments the treatment of each slice or area of the vaginal canal is performed twice, rotating the retractor 19 for instance by 45° around its own axis A-A between the first and the second treatment, thus avoiding discontinuities or irregularities in treating the tissue in correspondence of the edges of the pyramidal mirror 25, and in particular in correspondence of the bars 27. Rotating the retractor 19 only once by an angle different than 90° (in the illustrated example), for instance by 45°, there are no areas of the vaginal canal tissue remaining untreated or treated incompletely. A single rotation for each treatment depth is sufficient. It is also possible to perform a first treatment for the entire depth of the canal extracting the retractor 19 step-by-step and then to rotate the retractor 19 for instance by 45° and repeat the treatment for the areas not treated during the first phase, proceeding step-by-step whilst the retractor is inserted again, or vice versa in case the treatment has been started inserting the retractor step by step in the first phase.

The laser beam is advantageously pulsed and the movement thereof in the space is preferably controlled so as to treat adjacent but not overlapped tissue portions. In some embodiments the laser beam may be controlled to generate pulses with particular shape, for instance of the type described in WO2011096003.

Practically, the laser beam directed towards the reflecting surfaces 25A of the pyramidal mirror 25 can be controlled so as to involve volumes of vaginal canal tissue that are spaced from one another. Figure 9 schematically shows an example of a sequence of spots formed by the laser beam and indicated with L, which can be provided directing the laser beam against the mirror 25 and reflecting it by means of this mirror towards the wall of the vaginal canal. The laser beam involves for instance circular areas of tissue spaced from one another by areas not irradiated by the beam. Practically, the scanning mirrors 13A, 13B can control the movement of the laser beam so as to position it sequentially at each of the various points indicated in figure 9. In some embodiments the laser pulse may be synchronized with the movement of the mirrors 13A, 13B, so that the laser pulse is generated only when the mirrors are fixed in the position necessary to irradiate each single volume of the vaginal canal tissue. In some embodiments, the laser beam may be controlled so that in each position, defined by the scanning mirrors, more than one laser pulse is "shot" on the same tissue portion, for instance from two to four pulses.

Figure 9 schematically shows three rows of spots L generated by controlling the laser beam as described above. Each row is generated arranging the mirrors so as to direct the laser beam on the respective side reflecting surface 25A of the pyramidal mirror 25 at a substantially constant distance from the base edge. Subsequent rows are generated by moving the beam towards the vertex of the pyramid. Investing tissue volumes spaced from one another by means of the laser beam pulses in the spots L, the tissue recovery time is much shorter than with a continuous treatment or a treatment wherein the spots of the laser beam overlap so as to treat the whole surface of the vaginal canal.

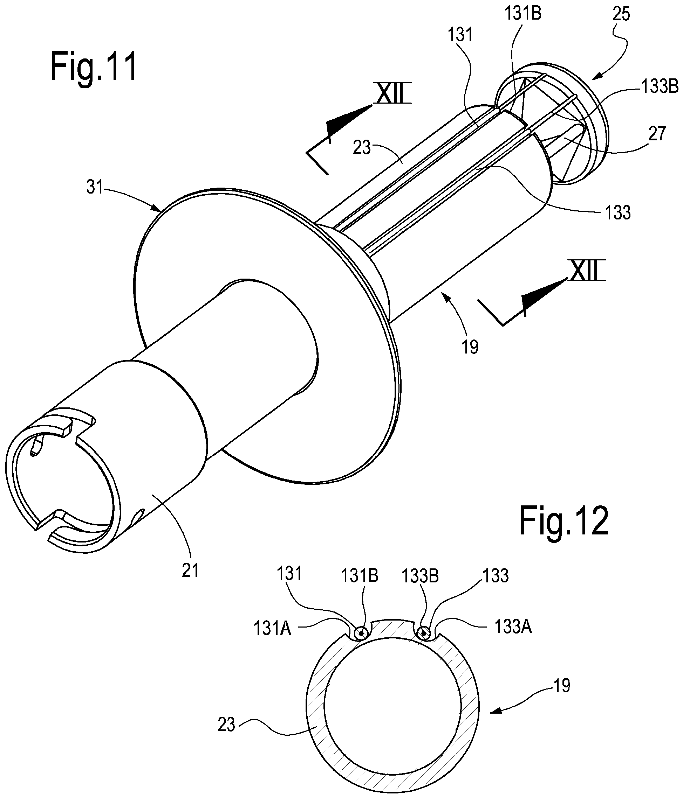

Characteristically, the device may be improved by including therein functionalities for radio-frequency (RF) treatment of the tissues forming the side wall of the vaginal canal and/or of other natural or surgically obtained orifices. Figures 11 and 12 show a possible configuration of electrodes for applying radio-frequency current.

In preferred embodiments at least one RF electrode and preferably two RF electrodes are arranged such as to surface on the retractor outer surface, which delimit the inner hollow volume of the retractor. In this manner the one or more RF electrodes make contact with the tissue of the vaginal canal (or other orifice), into which the retractor 19 has been introduced.

In some embodiments, as will be described in more detail herein after, the electrode(s) are electrically conductive towards the surrounding tissue in at least an area corresponding to or adjacent the aperture, window, opening or slit formed in the retractor 19 and where through the laser radiation exits the inner volume of the retractor 19 to irradiate the surrounding tissue.

Embodiments disclosed herein provide for electrodes extending parallel to the longitudinal axis A-A of retractor 19 on the cylindrical or substantially cylindrical body thereof.

In the embodiment of figures 11 and 12, two electrodes 131 and 133 may be applied on the outer wall of the cylindrical body 23 of the retractor 19. The electrodes 131 and 133 have preferably linear extension and extend for a part or the whole length of the cylindrical body 23. In some embodiments the electrodes 131 and 133 may be housed in grooves 131A and 133A (see figure 15) provided on the outer surface of the cylindrical body 23.

Advantageously, the electrodes 131 and 133 are parallel to each other and to the longitudinal axis of the cylindrical body 23 of the retractor 19.

In some embodiments, the two electrodes 131 and 133 are close to each other, i.e. they are arranged at the end of a limited arc, for instance a 45° arc, preferably 35° arc, and more preferably 30° or a smaller arc of the substantially circular development of the wall, of circular section, forming the cylindrical body 23. In vaginal applications, the distance between the electrodes 131 and 133 is such that it is possible to irradiate the areas adjacent to the clitoris and the urethra with RF currents. These RF currents have therapeutic effects in the treatment of incontinence in women. In practical embodiments of the treatment method, the retractor may be moved so that the irradiation with radio-frequency currents involves the whole vaginal canal, not only in correspondence of the clitoris but also in depth, in the areas adjacent to the bladder.

The two electrodes 131 and 133 can be connected, by means of suitable connectors (not shown), to the radio-frequency current source housed in the block schematically indicated with number 7. The laser and RF current sources can be controlled by the central unit 14, which may be programmed so as to timely coordinate the radio-frequency treatment and the laser treatment of the tissues. These two treatments can be consecutive, simultaneous or partially simultaneous, i.e. only partially overlapped. In some embodiments it is possible for instance to apply the radio-frequency current immediately before applying the laser and, if necessary, during the application of the laser.

In order to localize the radio-frequency treatment in the area where the laser beam is applied, according to advantageous embodiments the electrodes 131, 133 are insulated for a part of their longitudinal extension and are uncovered for instance in the area 131B, 133B in correspondence of the free space where the laser beam reflected by means of the mirror 25 passes. The portion of the electrodes 131, 133 housed in the seats or grooves 131A, 133A can be insulated. In this way the radio-frequency current is applied on tissues facing towards the pyramidal mirror 25, which can be therefore treated simultaneously with the laser radiation and the radio-frequency current.

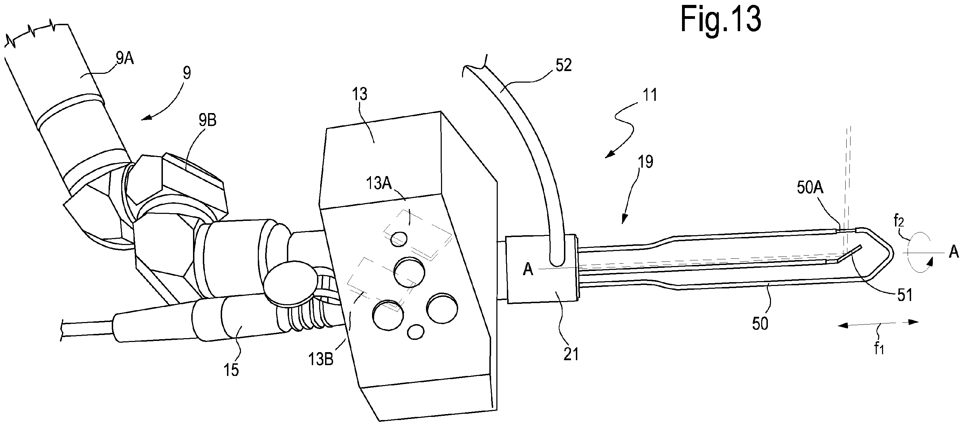

Figure 13 shows a simplified embodiment of the device 11. In this embodiment, the device 11 comprises again a box-shaped body forming a housing 13, inside which there are housed one or two scanning mirrors. Two scanning mirrors 13A and 13B are schematically shown with a broken line in the illustrated example. The movement of the mirrors around their axes of rotation is controlled by respective actuators, for instance galvanometers, under the control of a central unit 14, arranged for instance on the support 3 and connected to the device 11 via a cable 15. The control unit 14 is also connected to the laser source and to the RF current source, schematically indicated by means of the block 7, to control the emissions from these two sources. On the box-shaped body forming the housing 13 there may be buttons, capacitive sensors or other interface elements, allowing the user to handle the apparatus and to control the laser emission.

A retractor indicated again as a whole with number 19 is associated with the housing 13 of the device 11; this retractor can be advantageously applied to the housing 13 in reversible fashion, so that it is possible to use retractors 19 different in shape and dimension and/or to allow sterilization, or else to use disposable retractors 19 for hygiene and asepsis reasons.

In this embodiment the retractor 19 comprises elongated linear elements forming a sort of frame or grid defining inside itself a hollow space, wherein a system for directing the laser beam coming from the scanning system housed inside the housing 13 is housed. The hollow space is in this case bounded by a lattice-like or network structure formed by the elongated linear elements. In the illustrated embodiment the system for directing the laser beam comprises a mirror 51 inclined by about 45° with respect to the longitudinal axis A-A of the frame formed by the linear elements. This frame can be covered with a skirt, not shown, made for instance of plastic, to make the insertion thereof into the vaginal canal easier and to increase the patient's comfort. Advantageously, a preferably completely free window is provided in correspondence of the reflecting mirror 51.

One or preferably two of the linear elements forming the grid or frame, indicated with 50, comprise or are constituted by electrodes for the RF current coming from the source housed in the block 7. Reference number 52 indicates just by way of example a cable for the connection to the RF current source.

The electrode or electrodes 50 formed by the elongated linear elements of the retractor 19 are preferably insulated for a substantial part of their length. In advantageous embodiments they are uncovered only in the area 50A, in correspondence of the mirror 51. In this way, the laser radiation and the RF current can be applied in correspondence of the same area of tissue of the vaginal canal.

Inserting the retractor 19 in the vaginal canal, the mirror 23 takes such a position that the beam coming from the scanning system 13A, 13B is directed approximately orthogonal with respect to the axis A-A of the retractor, and therefore approximately orthogonal to a portion of the wall of the vaginal canal.

Moving the retractor according to the arrow f1 in a direction parallel to the axis A-A and rotating it according to the arrow f2 around the axis A-A, the operator can treat with the laser beam and the RF current adjacent and consecutive portions of the vaginal canal.

The laser beam is preferably a pulsed laser beam and, maintaining the mirror 51 fixed, each pulse is directed in a defined point by means of the scanning mirrors 13A, 13B. Maintaining the device 11 in a fixed position inside the vaginal canal for a given time, the device emits a series of pulses towards adjacent points arranged according to a pattern or path, predetermined by means of the programmable control unit 14 to treat the whole area that can be achieved while maintaining the mirror 51 in a given position. Once this treatment is ended, the retractor 19 can be angularly displaced to similarly irradiate all the tissue portions arranged at a given depth inside the vaginal canal. Once this treatment is ended, the retractor 19 can be moved according to the arrow f1 towards the inside or the outside (based upon where and how the treatment began), to repeat the operation on adjacent annular bands of the whole vaginal canal or of the areas to be treated.

In some applications, for instance for treating incontinence, the RF current can be applied only in a portion of the angular extension of the vaginal canal, as mentioned above with respect to the description of the embodiment of figures 11 and 12. In case the retractor 19 of figure 13 is used, when the whole angular extension of the vaginal canal shall be treated by means of the laser beam, rotating the retractor according to the arrow f2 it is possible to deactivate the RF source, so as to treat with this current only the desired area.

In the above description specific reference has been made to particularly advantageous embodiments for treating the vaginal canal. It should be however understood that a device of the type described above can be also used to treat tissues surrounding an orifice of different nature, for instance for treating the anal orifice, or an orifice provided surgically in a mass that is usually compact, i.e. usually devoid of orifices.

It is understood that the drawing only shows an example provided by way of a practical arrangement of the invention, which can vary in forms and arrangement without however departing from the scope of the concept underlying the invention. Any reference numerals in the appended claims are provided to facilitate reading of the claims with reference to the description and to the drawing, and do not limit the scope of protection represented by the claims.

## Claims

1. A device (1) for treating a vaginal canal or, either natural or surgically obtained orifices, by means of a laser beam, the device comprising:
- a retractor (19) for the wall of the vaginal canal or orifice, comprised of an outer wall surrounding a central hollow space, at least one aperture or window being provided for the laser beam directed from the hollow space of the retractor towards the exterior of the retractor;
- a laser directing system (25) configured and arranged for directing the laser beam towards said wall of vaginal canal or orifice and comprised of a mirror, arranged in said central hollow space;
- a laser beam scanning system (13), arranged and controlled for conveying the laser beam onto said mirror and moving said laser beam according to a predetermined path; **characterised by** further comprising - two electrodes (131, 133) for applying an RF current to the tissues of said wall of the vaginal canal or orifice, said two electrodes being at least partly arranged on the outer wall of the retractor; wherein the two electrodes extend at least across the at least one aperture or window of the retractor, such that laser radiation and radio frequency current can be applied on a same portion of tissue of the vaginal canal or orifice, into which the retractor has been introduced.

2. Device according to claim 1, wherein said two electrodes extend along a longitudinal extension of the retractor.

3. Device according to claim 3, wherein said two electrodes are approximately parallel to one another and to a longitudinal axis of the retractor.

4. The device according to claim 3, wherein the two electrodes are close to each other, at the end of an arc of 45°, preferably 35°, and more preferably 30° or less, of a substantially circular development of the outer wall, having a circular section, of a cylindrical body forming the retractor.

5. Device according to one or more of the previous claims, wherein said electrodes are insulated for a portion thereof and are not insulated in an area corresponding to the aperture or window where the laser beam is directed by means of said laser directing system.

6. Device according to one or more of the previous claims, wherein said retractor comprises retracting members defining a central hollow space inside which said mirror is arranged.

7. Device according to claim 6, wherein said retracting members comprise elongated linear elements, two of which constitute said two electrodes.

8. Device according to claim 6 or 7, wherein said mirror is arranged in a fixed position inside the space defined by the retracting members, near the distal end of said central hollow space.

9. Device according to claim 8, wherein said retracting members comprise a frame formed by elongated and shaped linear elements, two of which are associated with said two electrodes or are constituted by said two electrodes.

10. Device according to one or more of claims 1 to 6, wherein said mirror has a plurality of reflecting faces arranged around a longitudinal axis of the retractor.

11. Device according to claim 10, wherein said mirror is pyramid-shaped and has preferably flat side surfaces.

12. Device according to one or more of the previous claims, wherein said scanning system can be coupled to said retractor in a reversible manner.

13. An electro-medical apparatus comprising a laser source, a RF current source, and a device according to one or more of the previous claims connected with said laser source and with said RF current source.

## Patentansprüche

1. Vorrichtung (1) zur Behandlung eines Vaginalkanals oder von entweder natürlich oder chirurgisch erhaltenen Öffnungen mittels eines Laserstrahls, wobei die Vorrichtung aufweist:
einen Retraktor (19) für die Wand des Vaginalkanals oder der Öffnung mit einer Außenwand, die einen zentralen Hohlraum umgibt, mit einer Öffnung oder einem Fenster, das für den Laserstrahl vorgesehen ist, der von dem Hohlraum des Retraktors zu dem Äußeren des Retraktors gerichtet wird,
einem Laser-Richtsystem (25), das zum Richten des Laserstrahls zu der Wand des Vaginalkanals oder der Öffnung ausgebildet und angeordnet ist und einen Spiegel aufweist, der in dem zentralen Hohlraum angeordnet ist,
einem Laserstrahl-Abtastsystem (13), das zum Leiten des Laserstrahls auf den Spiegel und zum Bewegen des Laserstrahls entsprechend eines vorgegebenen Wegs angeordnet ist und gesteuert wird,
**gekennzeichnet durch** ferner zwei Elektroden (131, 133) zum Anlegen eines RF-Stroms an das Gewebe der Wand des Vaginalkanals oder der Öffnung, wobei die beiden Elektroden mindestens teilweise an der Außenwand des Retraktors angeordnet sind, wobei die beiden Elektroden sich mindestens über die mindestens eine Öffnung oder das Fenster des Retraktors erstrecken, sodass Laserstrahlung und Funkfrequenzstrom an denselben Teil des Gewebes des Vaginalkanals oder der Öffnung, in die der Retraktor eingebracht wurde, angelegt werden kann.

2. Vorrichtung nach Anspruch 1, wobei die beiden Elektroden sich entlang einer Längsausdehnung des Retraktors erstrecken.

3. Vorrichtung nach Anspruch 3, wobei die beiden Elektroden etwa parallel zueinander und zu einer Längsachse des Retraktors sind.

4. Vorrichtung nach Anspruch 3, wobei die Elektroden an dem Ende eines Bogens von 45°, vorzugsweise 35° und insbesondere vorzugsweise 30° oder weniger einer im Wesentlichen kreisförmigen Entwicklung der äußeren Wand, die einen kreisförmigen Querschnitt aufweist, eines zylindrischen Körpers, der den Retraktor bildet, nahe beieinander sind.

5. Vorrichtung nach einem oder mehreren der vorstehenden Ansprüche, wobei die Elektroden für einen Teil von ihnen in einem Bereich entsprechend der Öffnung oder dem Fenster, wo der Laserstrahl mittels des Laser-Rechtssystems gerichtet wird, nicht isoliert sind.

6. Vorrichtung nach einem oder mehreren der vorstehenden Ansprüche, wobei der Retraktor Rückzugselemente aufweist, die einen zentralen Hohlraum definieren, innerhalb dessen der Spiegel angeordnet ist.

7. Vorrichtung nach Anspruch 6, wobei die Rückzugselemente längliche lineare Elemente aufweisen, von denen zwei die beiden Elektroden bilden.

8. Vorrichtung nach Anspruch 6 oder 7, wobei der Spiegel in einer festen Position innerhalb des Raums, der durch die Rückzugselemente definiert wird, nahe des distalen Endes des zentralen Hohlraums angeordnet ist.

9. Vorrichtung nach Anspruch 8, wobei die Rückzugselemente einen Rahmen aufweisen, der durch längliche und geformte lineare Elemente gebildet ist, von denen zwei den beiden Elektroden zugeordnet sind oder durch die beiden Elektroden gebildet sind.

10. Vorrichtung nach einem oder mehreren der Ansprüche 1 bis 6, wobei der Spiegel eine Anzahl von Reflexionsflächen aufweist, die um eine Längsachse des Retraktors angeordnet sind.

11. Vorrichtung nach Anspruch 10, wobei der Spiegel pyramidenförmig ist und vorzugsweise flache Seitenflächen aufweist.

12. Vorrichtung nach einem oder mehreren der vorstehenden Ansprüche, wobei das Abtastsystem mit dem Retraktor in einer reversiblen Weise verbunden werden kann.

13. Elektro-medizinisches Gerät mit einer Laserquelle, einer RF-Stromquelle und einer Vorrichtung nach einem oder mehreren der vorstehenden Ansprüche, die mit der Laserquelle und mit der RF-Stromquelle verbunden ist.

## Revendications

1. Appareil (1) destiné à traiter un canal vaginal ou des orifices obtenus soit naturellement, soit chirurgicalement, au moyen d'un rayon laser, l'appareil comprenant :
- un rétracteur (19) pour la paroi du canal vaginal ou de l'orifice, comprenant une paroi externe entourant un espace central évidé, au moins une ouverture ou une fenêtre prévue pour le rayon laser, dirigée depuis l'espace évidé du rétracteur vers l'extérieur du rétracteur ;
- un système d'orientation du laser (25) conçu et disposé pour diriger le rayon laser vers ladite paroi de canal vaginal ou d'orifice et constitué d'un miroir, disposé dans ledit espace évidé central ;
- un système de balayage par rayon laser (13) disposé et contrôlé pour convoyer le rayon laser sur ledit miroir et déplacer le rayon laser selon un chemin prédéterminé ;
**caractérisé en ce qu'**il comprend en outre deux électrodes (131, 133) destinées à appliquer un courant RF aux tissus de ladite paroi de canal vaginal ou d'orifice, lesdites deux électrodes étant au moins partiellement disposées sur la paroi externe du rétracteur ;
les deux électrodes s'étendant au moins à travers l'au moins une ouverture ou fenêtre du rétracteur, de sorte que la radiation laser et le courant radiofréquence puissent être appliqués sur la même portion de tissu du canal vaginal ou de l'orifice où le rétracteur a été introduit.

2. Appareil selon la revendication 1, dans lequel lesdites deux électrodes s'étendent le long d'une extension longitudinale du rétracteur.

3. Appareil selon la revendication 3, dans lequel lesdites deux électrodes sont approximativement parallèles l'une par rapport à l'autre et à un axe longitudinal du rétracteur.

4. Appareil selon la revendication 3, dans lequel lesdites deux électrodes sont proches l'une de l'autre à l'extrémité d'un arc de 45°, de préférence de 35°, et plus préférentiellement de 30° ou moins, d'un développement substantiellement circulaire de la paroi externe de section circulaire d'un corps cylindrique formant le rétracteur.

5. Appareil selon au moins une des revendications précédentes, dans lequel lesdites électrodes sont isolées sur une de leurs portions et ne sont pas isolées sur une surface correspondant à l'ouverture ou la fenêtre par où le rayon laser est dirigée au moyen dudit système d'orientation du laser.

6. Appareil selon au moins une des revendications précédentes, dans lequel ledit rétracteur comprend des éléments rétractables définissant un espace creux central à l'intérieur duquel est disposé ledit miroir.

7. Appareil selon la revendication 6, dans lequel lesdits éléments rétractables comprennent des éléments linéaires allongés, dont deux constituent lesdites deux électrodes.

8. Appareil selon la revendication 6 ou 7, dans lequel ledit miroir est disposé dans une position fixe à l'intérieur de l'espace défini par les éléments rétractables, près de l'extrémité distale dudit espace évidé central.

9. Appareil selon la revendication 8, dans lequel lesdits éléments rétractables comprennent un cadre formé par des élément allongés et conformés linéaires, dont deux sont associés auxdites deux électrodes ou sont constitués desdites deux électrodes.

10. Appareil selon au moins une des revendications 1 à 6, dans lequel ledit miroir a plusieurs faces réfléchissantes disposées autour d'un axe longitudinal du rétracteur.

11. Appareil selon la revendication 10, dans lequel ledit miroir est de forme pyramidale et a, de préférence, des surfaces latérales planes.

12. Appareil selon au moins une des revendications précédentes, dans lequel ledit système de balayage peut être couplé audit rétracteur de façon réversible.

13. Appareil électromédical comprenant une source laser, une source de courant RF et un appareil selon au moins une des revendications précédentes connecté à ladite source laser et à ladite source de courant RF.
